Europäisches Patentamt

European Patent Office

Office européen des brevets

(11) Veröffentlichungsnummer: **0 274 706**

**A2**

(12) **EUROPÄISCHE PATENTANMELDUNG**

(21) Anmeldenummer: **87118780.3**

(22) Anmeldetag: **17.12.87**

(51) Int. Cl.⁴: **B65D 43/06** , B65F 1/16

(30) Priorität: **12.01.87 DE 3700683**

(43) Veröffentlichungstag der Anmeldung:
**20.07.88 Patentblatt 88/29**

(84) Benannte Vertragsstaaten:
**AT BE CH ES FR GB GR IT LI LU NL SE**

(71) Anmelder: **Gebrüder OTTO KG.**
**Siegener Strasse 69**
**D-5910 Kreuztal(DE)**

(72) Erfinder: **Otto, Werner, Ing.**
**Forstweg 2**
**D-5910 Kreuztal Krs. Siegen(DE)**
Erfinder: **Beese, Ulrich, Dipl.-Ing.**
**Brunnenstrasse 6**
**D-5963 Wenden-Hünsborn(DE)**
Erfinder: **Schmidt, Martin, Dipl.-Ing.**
**Am Zollstock 11**
**D-3565 Breidenbach-Achenbach(DE)**

(74) Vertreter: **Staeger, Sigurd, Dipl.-Ing.**
**Patentanwälte Dipl.-Ing. S. Staeger Dipl.-Ing.**
**Dipl.-Wirtsch.-Ing. R. Sperling Müllerstrasse**
**31**
**D-8000 München 5(DE)**

(54) **Einwegbehälter.**

(57) Ein Einwegbehälter mit einem Deckel ist mittels zweier verschiedener Verschlüsse eienrseits vorläufig und andererseits endgültig verschließbar, wobei der endgültige Verschluß nicht ohne Zerstörung des Verschlusses wieder zu öffnen ist. Der zu einem mehrmaligen Gebrauch geeignete vorläufige Verschluß (14) ist so ausgebildet, daß mit dem Verschließen gleichzeitig ein Anpressen einer en der Behälteraußenseite zwischen dem Behälterrand und dem Deckel vorgesehene Dichtung, bestehend aus einem Dichtungsring und einer umlaufenden Gegenfläche (11), zusammengepreßt wird.

FIG. 4

## Einwegbehälter

Die Erfindung betrifft einen Einwegbehälter, insbesondere für Krankenhausabfälle, mit einer mittels eines Deckels verschließbaren Einfüllöffnung, wobei der Deckel mittels eines axial zur Einfüllöffnung gerichteten Deckelrands den umlaufenden Rand der Einfüllöffnung überlappt, mit einem ohne Zerstörung nicht lösbaren Verschluß, bei dem eine am Deckelrand angeordnete Einrastkante einen Haltevorsprung am Behälter übergreift.

Da derartige Abfallbehälter während des Gebrauchs in Operationssälen oder in Krankenzimmern stehen, müssen sie in einem zumindest hygienisch einwandfreiem Zustand, das heißt in gewissem Maße keimfrei sein. Ein Sterilisieren von mehrmals benutzten Behältern ist kostenaufwendig und relativ umständlich. Daher verwendet man Einwegbehälter, die nach ihrer Füllung fest verschlossen werden und deren Entsorgung durch Verbrennen geschieht.

Aus der DE-OS 34 36 399 ist ein Einwegbehälter für Krankenhausabfälle bekannt, bei dem der Deckel mit seiner umlaufenden, nach unten gerichteten Nut auf die obere freie Randkante des Behälters aufgesetzt wird. An den sich gegenüberliegenden Nutwandungen ragen Vorsprünge hervor, zwischen denen sich der keilförmig ausgebildete Rand schiebt. Gleichzeitig wird der Deckel durch eine angeformte Dichtungslippe abgedichtet, die an einer Schrägfläche der Behälterinnenwand zur Anlage kommt.

Der ohne Zerstörung nicht mehr zu lösende Verschluß weist eine am Außenumfang des Randbereichs ausgebildete schräg nach unten gerichtete Rippe auf, deren freies Ende von einer entsprechenden Einrastkante des Deckels hintergriffen wird.

Dieser Behälter weist wesentliche Nachteile auf. Zum einen bietet der aufgesetzte Deckel, der lediglich den keilförmigen freien Rand der Behälteröffnung klemmt, keine Gewähr dafür, daß der Behälter, falls er während des Gebrauchs umfällt, verschlossen bleibt. Es steht zu befürchten, daß die Klemmwirkung zwischen der nach innen gerichteten Nut mit den darin angeformten Vorsprüngen und der keilförmig ausgebildeten Randkante der Einfüllöffnung nicht ausreicht, der durch das Umfallen erzeugten Erschütterung standzuhalten. Auch ist die am Behälterinnenrand angebracht Dichtlippe von Nachteil, da es leicht geschehen kann, daß bei einem gefüllten Behälter Abfallmaterial so weit hervorsteht, daß es sich unbemerkt zwischen diese Abdichtlippe und der Behälterinnenwand legt. Dadurch wird eine sichere Abdichtung gefährdet, da dann die Möglichkeit eines Austretens von Keimen besteht.

Der Erfindung liegt die Aufgabe zugrunde, einen Behälter der genannten Gattung so zu verbessern, daß er beim vorläufigen Verschließen stets einen sicheren Verschluß bietet und dessen Dichtung sowohl beim vorläufigen, als auch beim endgültigen Verschließen die Gefahr eines Einklemmens von Abfällen vermeidet.

Die Aufgabe wird erfindungsgemäß dadurch gelöst, daß der Dichtungsring am Außenumfang des Behälters im Randbereich unterhalb der Einfüllöffnung in eine umlaufende Nut aufgenommen ist, wobei eine an einem zugeordneten Abschnitt des Deckelrands ausgebildete umlaufende Gegenfläche gegen den Dichtungsring anpressbar ist und daß der Deckel zusätzlich mit einem zum mehrmaligen Gebrauch geeigneten Verschluß ausgebildet ist, der mit dem Verschließen gleichzeitig durch Anpressen der Gegenfläche gegen den Dichtungsring eine Abdichtung sicherstellt.

Durch die angegebenen Maßnahmen ist sichergestellt, daß ein Einklemmen von Abfallmaterial zwischen den zusammenwirkenden Dichtungselementen des Deckels und des Behälters ausgeschlossen wird, da die Bedienungsperson ein überhängen von Abfallmaterial aus der Einfüllöffnung heraus bemerkt und diesen Abfall in den Behälter zurückstopft. Das Ausbilden des mehrmals verwendbaren Verschlusses in Form eines Schraubverschluß stellt eine hohe Sicherheit gegen unbeabsichtigtes Öffnen bei einem eventuellen Umstürzen des Behälters dar; durch das Anpressen der Abstützfläche gegen den Dichtungsring in der Dichtungsnut beim vorläufigen Verschließen wird der Behälter geruchsdicht verschlossen und sichergestellt, daß aus einem umgestürzten Behälter keine Flüssigkeit austreten kann, da die Dichtung beim Verriegeln mittels des Drehverschlusses eine ausreichende Abdichtung sicherstellt. Zum endgültigen Verschließen wird der Deckel nach dem Aufschrauben durch festes Aufdrücken arretiert. Der dabei aufzubringende Druck auf den Deckel ist geringer als beim Stand der Technik, da durch das Aufschrauben bereits ein Teildruck aufgebracht ist. Somit wird das endgültige Verschließen durch die erfindungsgemäßen Merkmale erleichtert.

Vorteilhafterweise kann vorgesehen sein, daß die Nut seitlich am Außenumfang des Behälters mit radial nach außen gerichteter Öffnung angeordnet ist, wobei die untere Nutwand radial über der oberen Nutwand herausragt. Hier ist es günstig, daß die Gegenfläche im wesentlichen als Schrägfläche ausgebildet ist.

Vorteilhafterweise erstreckt sich die radial innenliegende Nutwand der Innenut in etwa bis zur Höhe der Schrägfläche bzw. der Nut für den Dichtungsring. Diese Nutwand gerät in Anlage an die konvex nach innen in den Behälter vortretende Nutwand für den Dichtungsring und bilet somit zum einen ein Widerlager für den Pressvorgang beim Abdichten und zum anderen eine selbständige zusätzliche Dichtung.

In den weiteren Unteransprüchen sind vorteilhafte Ausgestaltungen angegeben.

Im folgenden wird die Erfindung anhand in der Zeichnung dargestellter Ausführungsbeispiele näher erläutert.

Es zeigen:

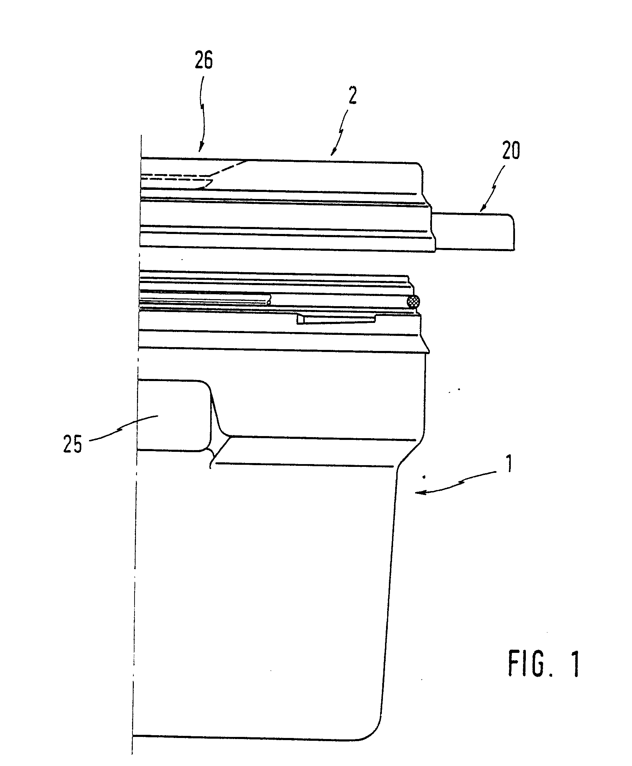

. Fig. 1 eine Teildarstellung einer Seitenansicht des erfindungsgemäßen Einwegbehälters mit Deckel,

Fig. 2 eine vergrößerte Teildarstellung einer Seitenansicht des Randbereichs der Einfüllöffnung,

Fig. 3 eine Teildarstellung eines Axialschnitts durch den Deckel,

Fig. 4 eine Teildarstellung des Behälters mit aufgesetztem Deckel,

Fig. 5A, 5B, Detaildarstellung der rampenartigen Vorsprünge des Drehverschlusses,

Fig. 6 eine weitere Darstellung eines Axialschnitts durch den Deckel.

In Fig. 1 ist ein Einwegbehälter 1 mit einem Deckel 2 dargestellt. Der Deckel besitzt einen radial abstehenden Handgriff 20 und an seiner vertieften Oberseite einen weiteren Handgriff 26. Am Behälter sind sich diametral gegenüberliegend Griffe 25 ausgeformt. Der in Fig. 1. dargestellte Behälter hat ein Volumen von etwa 30 Liter Die Erfindung ist jedoch auch auf Behälter mit einem höheren Volumeninhalt, z.B. 60 Liter anwendbar.

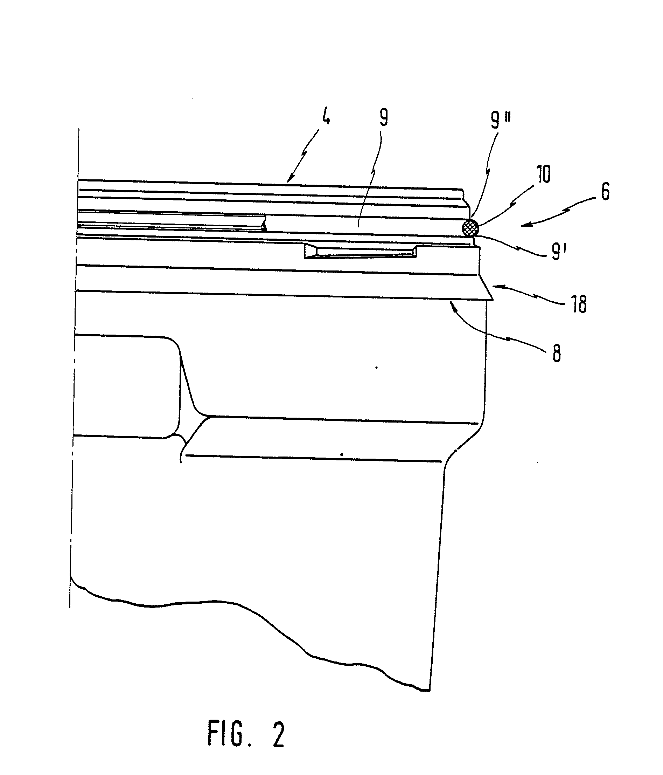

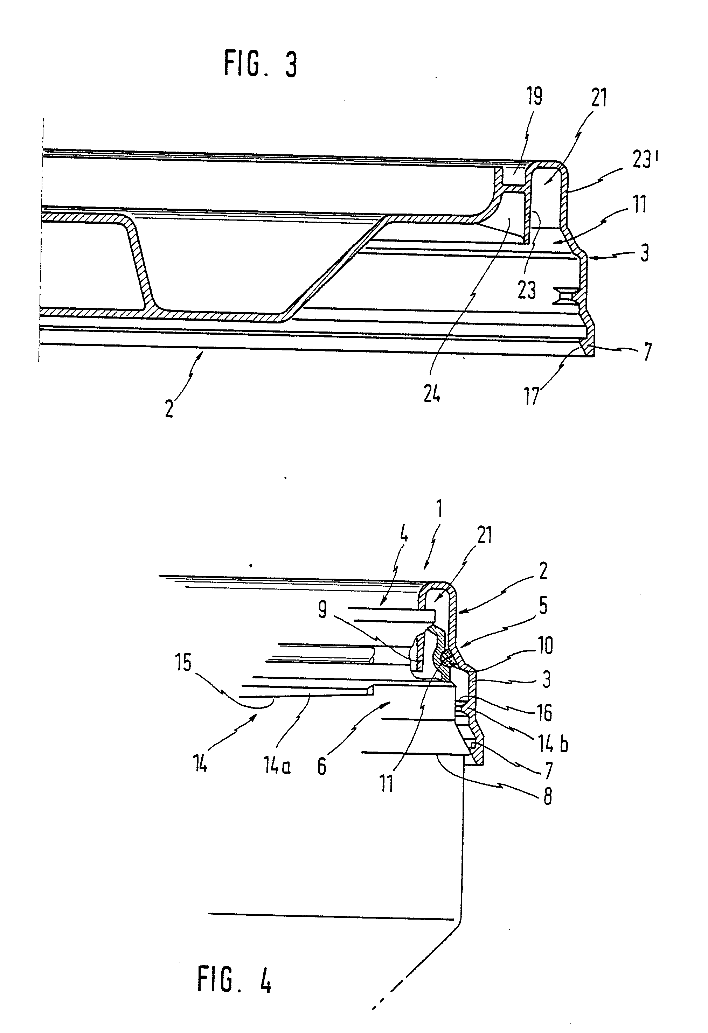

In der in Fig. 2 gezeigten Darstellung ist mit dem Bezugszeichen 4 die Einfüllöffnung gekennzeichnet. Im Randbereich 4 unterhalb der Einfüllöffnung 4 ist eine umlaufende Nut 9 angeformt. Die Nut ist derart ausgebildet, daß der behälterseitige Abschnitt 9' radial weiter nach außen vortritt, als der zur Einfüllöffnung nach oben gerichtete Abschnitt 9". Durch diese besondere Ausführung erhält man eine zusätzliche Abstützung für den Dichtungsring 10, der in die Nut eingesetzt ist. Der Dichtungsring wirkt mit einer Gegenfläche 5 zusammen, die an entsprechender Stelle am Deckel 2 ausgebildet ist. Diese Gegenfläche 11 ist im vorliegenden Ausführungsbeispiel als Schrägfläche ausgebildet. Beim Schließen des Deckels kommt diese Schrägfläche zur Anlage gegen den Dichtungsring 10 und drückt mit einer im wesentlichen schräg zur Achse des Behälters nach unten gerichteten Kraftkomponente den Dichtungsring 10 in die Dichtungsnut 9. (Vergl. Fig.4).

Unterhalb der in Fig. 4 insgesamt mit der Bezugsziffer 5 gekennzeichneten Dichtung ist ein Drehverschluß 14 ausgebildet. Dieser Drehverschluß 14 besteht aus zum einen am Behälter angeordneten rampenartigen Vorsprüngen 14A mit einer nach unten gerichteten Rampenfläche 15 und zum anderen aus am Deckel an entsprechender Stelle angeformten passend dazu ausgebildeten Vorsprüngen 14B mit nach oben gerichteten Rampenflächen 16. Diese Vorsprünge treten beim Verdrehen des Deckels nach Art von Gewindeflanken gegenseitig in Eingriff und erzeugen eine axial gerichtete Kraft, die zu einer Pressung der Dichtung 5 führt. Die Steigung der Rampenflächen 15, 16 ist dabei derart gewählt, daß ein Überdrehen des Verschlusses nicht möglich ist. Um dieser Gefahr zu begegnen, kann auch am entsprechenden Ende einer Rampenfläche eines Vorsprungs 16 oder eines Vorsprungs 15 eine Anschlagfläche vorgesehen sein, (nicht dargestellt).

Am freien Ende des axial zum Behälter gerichteten Deckelrands 3 ist nach Art eines Hakens eine Einrastkante 7 mit einer schrägen Gleitfläche 17 ausgebildet. Diese Einrastkante 7 hintergreift beim endgültigen Verschließen des Behälters einen unmittelbar am Außenumfang der Seitenwand des Behälters angeformten Haltevorsprung 8, wobei ein erhöhter Kraftaufwand erforderlich ist, damit die Einrastkante 7 durch Überspringen des Haltevorsprungs 8 in die Verschlußstellung gebracht werden kann. Dieser Kraftaufwand dient zum einen zum Zusammenpressen der Dichtung und zum anderen dem elastischen Aufweiten des freien Rands des Deckels 3, der, nachdem die Einrastkante 7 den Haltevorsprung 8 am Deckel übersprungen hat, in seine ursprüngliche Stellung zurückkehrt.Das Überspringen wird erleichtert durch eine Gleitfläche 18, die beim Aufdrücken des Deckels auf den Behälter mit der Gleitfläche 17 am Einrastvorsprung zusammenwirkt.

Dieser Verschluß ist ohne zerstörende Gewaltanwendung nicht wieder zu lösen. Eine weitere Erschwernis kann dadurch hergestellt werden, daß die Gleitfläche 17 nicht als eine Schrägfläche, sondern als eine parallel zur Wand unterhalb des Haltevorsprungs 8 ausgebildete Fläche ist, so daß eine nahezu hermetische und spaltenfreie Verriegelung erzielt wird (nicht dargestellt).

An der Deckelunterseite ist eine umlaufende Nut 21 ausgebildet, in die der freie Rand 22 der Einfüllöffnung 4 eintreten kann. Die radial innenliegende Nutwand 23 der Innennut 21 ist so weit heruntergezogen, daß sie sich bis zur Höhe der gegenüberliegenden Schrägfläche 11 erstreckt, welche letztere sich an die radial außenliegende Nutwand 23' nach unten anschließt. Die innenliegende Nutwand 23 ist mittels Stützrippen 24 abgestützt, wobei diese Stützrippen 24 gleichzeitig die Stabilisierung einer auf der Deckeloberfläche umlaufenden Stapelnut 19 sicherstellen.

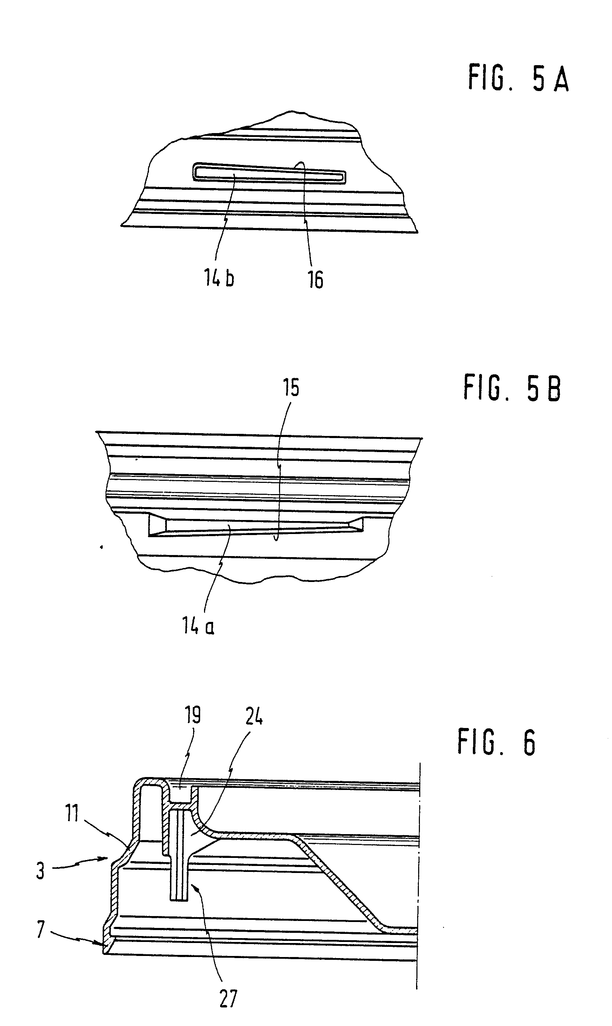

Durch die Anlage der radial innenliegenden Nutenwand 23 an der radial nach innen vortretenden Wand der Dichtungsnut 9 wird einerseits eine Abstützung für die Dichtung 5 und andererseits eine selbständige weitere Abdichtung bereitgestellt. In Fig. 6 ist ein weiteres Detail des Deckels dargestellt. Zumindest an vier, regelmäßig im Abstand auf dem Umfang verteilten Abstützrippen 24 sind axiale Vorsprünge 27 ausgebildet, die einer Zentrierung des Deckels auf der Einfüllöffnung 4 dienen.

Bei einer nicht dargestellten Ausführungsvariante kann vorgesehen sein, daß die Schrägfläche als eine gekrümmte, an einen Dichtungsring mehr oder weniger formgetreu angepaßte konkave Fläche ausgebildet ist.

## Ansprüche

1. Einwegbehälter, insbesondere für Krankenhausabfälle, mit einer mittels eines Deckels verschließbaren Einfüllöffnung, wobei der Deckel mittels eines axial zur Einfüllöffnung gerichteten Deckelrands den umlaufenden Rand der Einfüllöffnung überlappe, mit einem ohne Zerstörung nicht lösbaren Verschluß, bei dem eine am Deckelrand angeordnete Einrastkante einen Haltevorsprung am Behälter übergreift, dadurch **gekennzeichnet,** daß der Dichtungsring (10) am Außenumfang des Behälters (1) im Randbereich (6) unterhalb der Einfüllöffnung (4) in einer unlaufende Nut (9) aufgenommen und eine an einem zugeordneten Abschnitt des Deckelrands (3) ausgebildete umlaufende Gegenfläche (11) gegen die Dichtungsring (10) anpressbar ist und daß der Deckel (2) zusätzlich mit einem zum mehrmaligen Gebrauch geeigneten Verschluß (14) ausgebildet ist, der mit dem Verschließen gleichzeitig durch Anpressen der Gegenfläche (11) gegen den Dichtungsring (10) eine Abdichtung sicherstellt.

2. Einwegbehälter nach Anspruch 1, dadurch **gekennzeichnet,** daß die Nut (9) seitlich am Außenumfang des Behälters mit radial nach außen gerichteter Öffnung angeordnet ist, wobei die untere Nutwand (9') radial über der oberen Nutwand (9") herausragt.

3. Einwegbehälter nach Anspruch 1 oder 2, dadurch **gekennzeichnet,** daß die Gegenfläche (11) im wesentlichen als Schrägfläche ausgebildet ist.

4. Einwegbehälter nach mindestens einem der Ansprüche 1 - 3 , mit einer an der Unterseite des Deckels umlaufenden Innennut, in die der obere Abschnitt der Einfüllöffnung eingreift, dadurch **gekennzeichnet,** daß die radial innenliegende Nutwand (23) der Innennut (21) sich bis zur Höhe der Gegenfläche (11) bzw. der Nut (9) erstreckt und im Behälterinneren durch Anlage an einen umlaufenden Innenwandabschnitt ein Widerlager und eine weitere Dichtung bildet.

5. Einwegbehälter nach Anspruch 4 und mit einer auf der Oberseite des Deckels ausgebildeten und auf der Innenseite des Deckels mittels Rippen gestützten Stapelnut, dadurch **gekennzeichnet,** daß die Nutwand (22) mittels der sich radial erstreckenden Stützrippen (24) abgestützt ist.

6. Einwegbehälter nach mindestens einem der Ansprüche 1 bis 5, dadurch **gekennzeichnet,** daß der Drehverschluß (14) aus jeweils aneinander zugeordneten Abschnitten des Deckelrandes (3) bzw. des Randbereichs (6) der Einfüllöffnung (4) angeformten und regelmäßig über den Umfang verteilten rampenartigen Vorsprüngen (14A,14B) besteht, deren entsprechende Rampenflächen (15,16) nach Art von Gewindeflanken gegenseitig zur Anlage bringbar sind.

7. Einwegbehälter nach mindestens einem der Ansprüche 1 - 6, dadurch **gekennzeichnet,** daß am Deckelrand (3) radial nach außen abstehend ein Handgriff (20) angeformt ist.

8. Einwegbehälter nach mindestens einem der Ansprüche 1 - 7, dadurch **gekennzeichnet,** daß an der Deckelunterseite axial vortretende Führungsvorsprünge (24) angeformt sind, die am Innenrand der Einfüllöffnung (4) in den Behälter hineinragen.

9. Einwegbehälter nach mindestens einem der Ansprüche 1 - 8, dadurch **gekennzeichnet,** daß der behälterseitige Vorsprung (8) des nicht lösbaren Verschlusses (7,8) unmittelbar am Außenumfang des Randbereichs (6) der Einfüllöffnung (4) angeformt ist.

10. Einwegbehälter nach mindestens einem der Ansprüche 1 - 9, dadurch **gekennzeichnet,** daß die den Dichtungsring (10) aufweisende Dichtung zwischen dem Drehverschluß und der oberen Randkante der Einfüllöffnung (4) angeordnet ist.

FIG. 1

FIG. 2

# FIG. 3

# FIG. 4

FIG. 5 A

14 b     16

FIG. 5 B

15

14 a

FIG. 6

19     24

11

3

7

27